# EUROPEAN PATENT SPECIFICATION

(11) **EP 0 274 246 B1**
(45) Date of publication and mention of the grant of the patent: **17.03.1993**
(21) Application number: 87310957.3
(22) Date of filing: 14.12.1987
(51) Int. Cl.: G01F 1/00, B63C 11/24, A61M 16/00

(54) **Measuring apparatus**
Messgerät
Appareil de mesure

(30) Priority: 13.12.1986 GB 8629825; 02.04.1987 GB 8707869
(43) Date of publication of application: 13.07.1988
(73) Proprietor: J Downs (Jersey) Limited, St. Helier Jersey Channel Islands (GB)
(72) Inventor: Jones, Gareth Telfer, Altens Aberdeen (GB); McCormick, James, Howe Moss Drive Dyce Aberdeen (GB)
(74) Representative: Fitzpatrick, Alan James

(56) References cited:
- US-A- 3 429 796
- US-A- 3 593 735
- US-A- 3 767 552
- US-A- 3 967 920
- US-A- 4 187 721
- US-A- 4 233 842
- PATENT ABSTRACTS OF JAPAN, vol. 9, no. 292 (P-406)[2015], 19th November 1985;& JP-A-60 129 642

## Description

This invention relates to gas flow measuring systems, particularly systems for measuring volumetric gas flow rates preferably also total volumetric gas flow.

There are circumstances where it is desirable or essential to know instantaneous or short-term volumetric gas flow rates, and preferably also the total volume of gas transferred in the longer term or over a predetermined period of time. For example, the monitoring of breathing gas mixture consumption rates in life support systems can give indications of metabolic rates and system leaks, while a measurement of total volumetric gas flow can be employed for determining overall efficiency and total cost of consumed gases.

Inspection, maintenance, and repair operations in the offshore oil industry may require divers to operate for extended periods of time in water of great depth. For well known reasons, divers working at great depth and correspondingly great pressure are not permitted to breathe ordinary air. Consequently, such divers must be supplied with a mixture of helium and oxygen, the correct percentage of oxygen being a function of the diver's operating depth.

Helium and oxygen of the necessary purity and in the substantial amounts needed for diving operations (including preceding preparation of the diver and succeeding depressurisation) are very expensive. Helium consumption is minimised by supplying the diver through a closed-loop breathing system in which the exhaled gases are passed through a reprocessing system which removes carbon dioxide (and preferably also moisture and other vapours) and replaces metabolic oxygen, while leaving the helium content theoretically intact.

Such closed loop breathing systems are known, but the retention of helium is almost inevitably less than perfect. Loss of helium also occurs through leakage, which will be exacerbated by the hyperbaric operating pressures, and poorly maintained equipment.

As mentioned already, the correct proportion of oxygen in the helium/oxygen mixture is not fixed, but is a function of operating conditions. Further complications in maintenance of the correct proportions of helium and oxygen arise from the injection of make-up gases into the system to compensate for leaks and for any necessary increase in system pressure. It is desirable, for reasons including efficiency monitoring and cost control, to know the total volumetric flows that have occured over the entire diving operation (or any distinct stage of the diving operation) since these flow totals can give indications of the efficiency of various parts of the system and the financial costs of the gases. It is also clearly desirable to have an accurate measure of the total volume of bulk gases delivered by a gas manufacturer/supplier to the gas user (in a manner analogous to the metering of bulk supplies of liquid fuels). Such measurements are not at present readily obtainable with acceptable accuracy due to the limitations of known forms of gas flow measuring equipment

It is an object of the invention to provide a gas flow measuring system for measuring volumetric gas flow rates, and which may be adapted to measure total volumetric gas flow.

According to the present invention there is provided a gas flow measuring system comprising a gas flow conduit for carrying a flowing gas mixture of known constituents in varying proportions, a gas analyser coupled to the conduit whereby the specific gravity of the flowing gas mixture may be determined, a mass flowmeter coupled to the conduit for measuring the mass flow rate of the flowing gas mixture, and a measurement correlator for correlating a currently determined specific gravity of the mixture and the mass flow rate to produce a resultant signal representative of the volumetric flow rate of the flowing gas mixture.

The flowing gas mixture is preferably a binary gas mixture, for example a mixture of helium and oxygen free of other gases in quantities sufficient to cause substantial errors in the measurements.

Other binary gas mixtures may consist of oxygen-enriched air, i.e. nitrogen and oxygen with the oxygen in excess of 21 volume percent (for therapeutic purposes), and anaesthetic/analgesic gas mixtures such as nitrous oxide and oxygen. Alternatively, the flowing gas mixture may contain more than two components, provided that the components can be allocated to two groups such that the components within each group remain in mutually fixed proportions and only the proportion of one group in toto varies with respect to the other group in toto as respective fractions of the overall mixture. In addition to breathing gas mixtures, the flowing gas mixture may comprise one or more components intended for industrial processes such as chemical engineering. It should further be noted that multiple mixtures of independently variable gases may be measured provided that a suitable gas analyser is provided for each additional gas in the mixture.

The gas analyser is preferably an electrochemical gas analyser as described in either of United States Patents 3,429,796 and 3,767,552. Such analysers are particularly suited to the measurement of oxygen content of gas mixtures, and can readily determine the proportion of oxygen in a helium/oxygen mixture to produce a representative electrical output signal. Since the specific gravities of pure helium and of pure oxygen are each known (at standardised pressure and temperature), measurement of the oxygen content of a helium/oxygen mixture can be directly converted to a specific gravity reading. Such a gas analyser is preferably calibrated against a gas "mixture" which in practice will be pure helium, but any suitable gas mixture of known proportions can be employed for calibration. (If the non-oxygen component(s) of the gas mixture is not helium, then the appropriate gas would be used for calibration, such as nitrogen for subsequent analysis of oxygen-enriched air).

The mass flowmeter is preferably a Coriolis force mass flowmeter as described in United Kingdom Patent 2,001,759, which is capable of directly measuring mass flow rate through a conduit to produce a representative electrical output signal. Another Coriolis-type mass flowmeter is described in United States Patent 4,691,578, and this flowmeter also produces an electrical output signal which is representative of the mass flow rate of fluid flowing through a conduit.

JP-A-60 129 642 discloses the use of two flowmeters wherein the flow measured by the first is used with the output of the second to determine composition of the flow. US-A-3 593 735 discloses a gas analyser in a system for maintaining a desired oxygen partial pressure in breathing gas supplied to a diver.

The measurement correlator generally operates by dividing the mass flow rate measurement by the density of the mixture (obtained from the specific gravity measurement) to give the required volumetric flow rate measurement (the relationship between mass and volumetric flow rates for a compressible fluid is non-linear, and the correlation must take account of this non-linearity). In the case where the specific gravity measurement is made directly or indirectly (e.g. from oxygen content measurement) at the operating pressure of the system, the specific gravity measurement will pertain to the gas pressure within the gas flow conduit, which may be substantially above atmospheric pressure when the flowing gas mixture is a breathing mixture for a diver. Consequently, the volumetric flow rate measurement will be an actual volumetric flow rate pertaining to the actual pressure and temperature at the location where the specific gravity is measured. This actual volumetric flow rate measurement is preferably also presented as an equivalent volumetric flow rate at a standardised pressure and temperature, suitably 1 bar or nominal atmospheric pressure at sea level, and zero degrees Celsius. Conversion of the volumetric flow rate measurement is achieved by measuring the ratios of actual gas pressure and temperature in the gas flow conduit to the standardised pressure and temperature, and multiplying the actual flow rate measurement by these ratios or by other suitably related factors. Measurement of actual gas pressure and temperature can be performed by any suitable devices or equipment, which may be an integral part of the gas analyser, or of the mass flowmeter, or separate therefrom.

A potentially simpler procedure for obtaining the equivalent volumetric flow rate at standardised conditions is to make the specific gravity measurement under these standardised conditions. Thus in the case where the gas analyser functions by analysing oxygen content, gas samples can be obtained by bleeding a small but representative flow of gas mixture from the gas conduit through a pressure regulator or other pressure reducing device, and presenting the sampled gases to the gas analyser substantially at the standardised pressure, and at a temperature which does not differ from the standardised temperature by an amount which will produce significant errors in the resultant analytical measurements.

Measurement of volumetric flow rate can be converted to a measurement of total volumetic flow in a given period of time by integration of the instantaneous volumetric flow rate measurements throughout that period of time. Integration of the appropriate electrical signals can be carried out by any suitable analogue and/or digital electronic circuit. Indications of total volumetric flow may be given both for actual volume, and for equivalent volume at the standardised pressure and temperature.

In the case where the gas flow measuring system is applied to measuring the volumetric flow rate of a breathing gas mixture supplied to a diver in a closed-loop breathing system into which a make-up gas mixture may be injected, the gas flow measuring system is preferably duplicated and the second gas flow measuring system is coupled in series with the make-up gas mixture supply such that the volumetric flow rate and composition of the injected make-up mixture can be measured in like manner to the gas mixture flowing around the closed loop.

The make-up gas mixture can be injected into the closed loop upstream or downstream of the system applied to measuring the gasflow around the closed loop.

Embodiments of the present invention will now be described by way of example, with reference to the accompanying drawings wherein:-
Fig. 1 is a schematic circuit diagram of a closed loop breathing gas mixture supply system incorporating a first embodiment of a gas flow measuring system in accordance with the invention.
Fig. 2 is a schematic circuit diagram of a second embodiment of gas flow measuring system in accordance with the invention, in the form of a modification of the first embodiment; and
Fig. 3 is a schematic circuit diagram of a modified form of gas analyser suitable for substitution into the first and second embodiments of the invention.

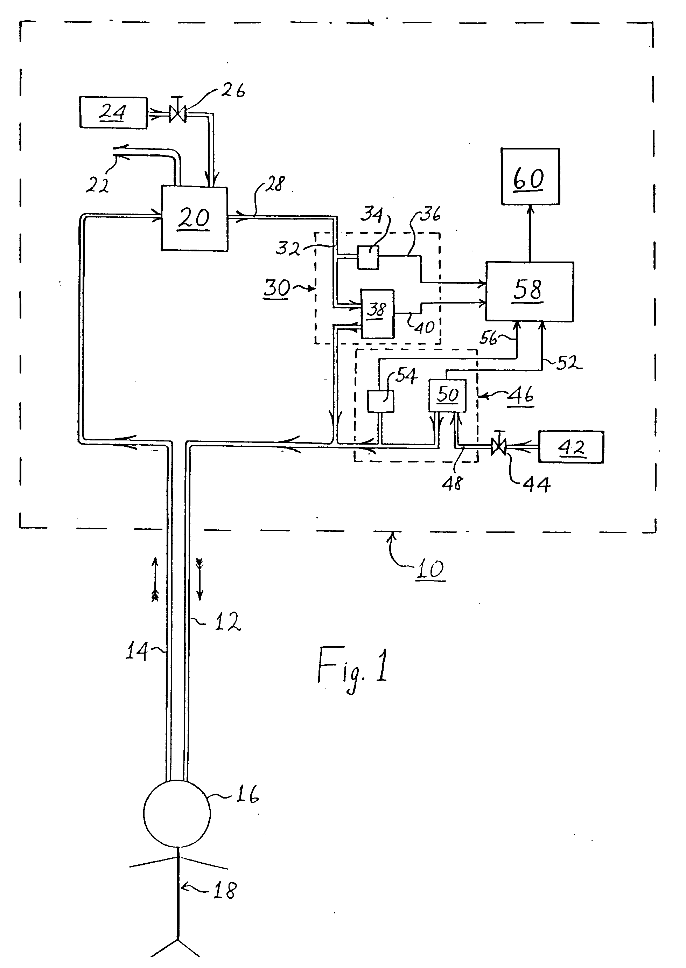

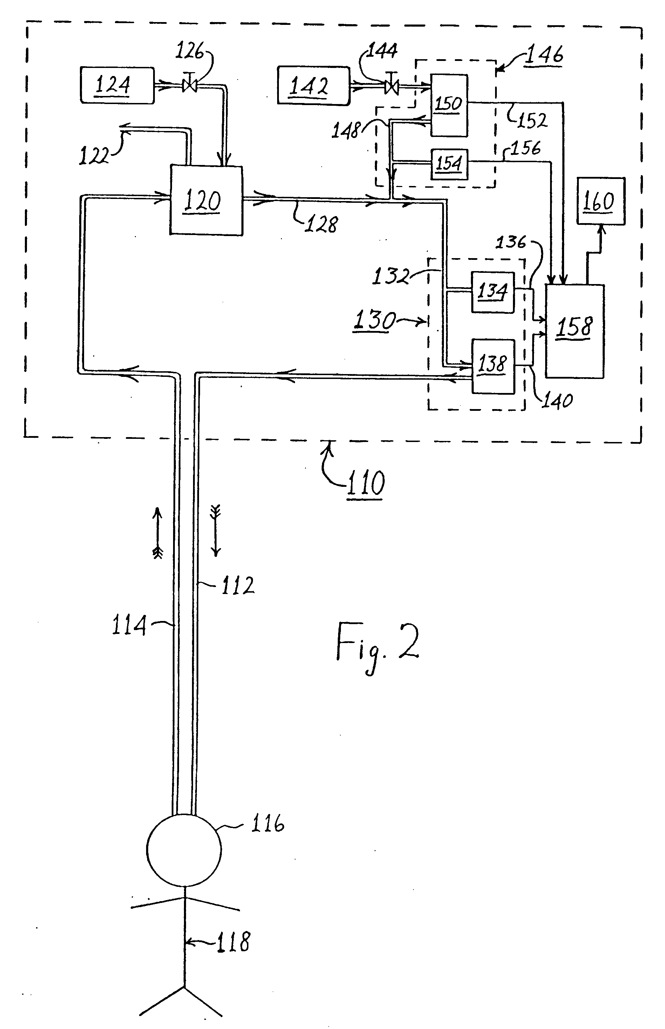

Figs.1 and 2 are both highly schematic diagrams showing functional blocks interconnected by gas conduits and electrical connections.

In Fig. 1, a diver's breathing gas mixture supply system 10 is mounted on a ship or oil rig (not shown) and is coupled through a supply hose 12 and a return hose 14 to the helmet 16 of a diver 18. The breathing gas mixture is helium and oxygen, with 1 - 21 volume percent of oxygen according to requirements. In normal operation, the system 10 is a closed loop breathing system in which exhaled breathing gases are returned from the helmet 16 via the return hose 14 to a reprocessing system 20. Within the reprocessing system 20, carbon dioxide is removed from the exhaled gases by any suitable method, and either vented through a waste gas exhaust 22 or internally absorbed in a cannister of soda lime (not shown). Since the oxygen content of the breathing mixture is depleted by breathing, the proportion of oxygen is returned to the correct level by injection from an oxygen supply 24 through a control valve 26. The reprocessing system 20 preferably also regulates the humidity of the regenerated breathing gas mixture delivered at its output conduit 28; temperature may additionally be regulated.

Before the output of the reprocessing system 20 is fed back into the diver's supply hose 12, the regenerated gas mixture has its volumeric flow rate measured in a first gas flow measuring system 30 which has a gas flow conduit 32 coupled to the regenerator output conduit 28. A gas analyser 34 is coupled to the gas flow conduit 32, to measure the oxygen content of the helium/oxgen mixture and to produce a representative electrical output signal on an output signal lead 36. The gas analyser 34 is an electrochemical oxygen analyser as described in either of United States Patents Nos. 3,429,796 and 3,767,552.

The gas flow conduit 32 within the first measuring system 30 is next coupled to a mass flowmeter 38 which is a Coriolis-type mass flowmeter. Meters of this type operate by passing the flowing gas through a curved conduit which is cantilever mounted and oscillated at right angles to the plane of curvature such that Coriolis forces induced by gas flowing in the conduit induce phase differences in the vibratory motions of different parts of the conduit. These phase differences are dependent on the mass flow rate of gas in the curved conduit so that measurement of the phase differences gives a measurement of the mass flow rate. The very small phase differences induced by the relatively low mass flow of a gas mixture predominantly consisting of helium mean that high accuracy is required in detecting and amplifying the output signal. Full details of the preferred form of the mass flowmeter 38 are given in United Kingdom Patent No. 2,001,759, and it may be constituted by the commercially available "Micromotion D12 meter". In operation, the mass flowmeter 38 produces an electrical output signal representative of the mass flow rate on an output signal lead 40.

The continuation of the gas flow conduit 32 beyond the mass flowmeter 38 conveys the flow-measured gas mixture out of the first flow measuring system 30 and into the surface end of the diver's supply hose 12.

Although the breathing system described above is nominally a closed loop system, there will in practice be a need to add gases to the system, either intermittently or continuously. The need arises from a number of causes, including leaks, deliberate venting or blowdown, and general increases in system pressure (for example, due to descent of the diver 18). The make-up gases are supplied as required from a pressurised gas source 42 containing a helium/oxygen mixture of known proportions. Make-up gas supply from the gas source 42 is regulated by a control valve 44. For example, if the diver 18 is operating at a depth of 100 metres at which the ambient water pressure is about 10 bar, the pressure required at the inlet end of the supply hose 12 is about 18 bar, and the pressure of make-up gases in the gas flow conduit 48 will be maintained at the same 18 bar pressure to match the pressure of regenerated breathing gas mixture in the gasflow conduit 32.

The volumetic flow rate of the make-up gases from the gas source 42 is measured by passing the make-up gases through a second gas flow measuring system 46. The flow measuring system 46 is similar to, and may be identical to, the first gas flow measuring system 30.

The supply of make-up gases released through the control valve 44 enters a gap flow conduit 48 passing completely through the flow measuring system 46. The gas flow conduit 48 first carries the make-up gases through a Coriolis-type mass flowmeter 50 which is similar or identical to the mass flowmeter 38. In operation, the mass flowmeter 50 produces a mass-flow-representative electrical output signal on an output signal lead 52. The gas flow conduit 48 conveys the make-up gases from the mass flowmeter to a gas analyser 54 which measures the oxygen content of the make-up gases to produce a representative electrical output signal on an output signal lead 56. The make-up gases then leave the second flow measuring system 46 and enter the surface end of the supply hose 12 at a point immediately downstream of the first flow measuring system 30 (which is delivering the regenerated breathing gas mixture to the inlet or surface end of the supply hose 12).

The output signal leads 36, 40, 52 and 56 are each connected to a measurement correlator 58. Prior to operational use, the supply system 10 will have been calibrated by supplying pure helium (or a helium/oxygen mixture of known proportions) at a suitable known pressure and temperature to the gas analysers 34 and 54, to cause calibration measurements to be produced and fed to the correlator 58 along the leads 36 and 56. The correlator 58 is thereby enabled during operational use to convert the oxygen content measurement signals on the leads 36 and 56 to equivalent specific gravity measurement signals for the gas mixtures in the gas flow conduits 32 and 48 respectively.

The correlator 58 also receives direct mass flow rate measurements via the leads 40 and 52 from the mass flowmeters 38 and 50 respectively.

From these indirect specific gravity measurements and the direct mass flow measurements, the correlator 58 can correlate the measurements to produce respective actual volumetric flow rates in the gas flow conduits 32 and 48, and by further correlation, the volumetric flow rate of the gases entering the supply hose 12. These various volumetric flow rates can be individually or collectively indicated on a suitable display device 60 adjacent to the measurement correlator 58 and/or at a separate diving control station (not shown).

Pressure and temperature measurements may be taken by suitable transducers (not illustrated) either incorporated in the gas analysers 34 and 54 or otherwise respectively coupled to the gas flow conduits 32 and 48, to feed respective pressure and temperature measurement signals to the correlator 58. Such pressure and temperature measurements can enable the correlator 58 to convert the actual volumetric flow rates to equivalent volumetric flow rates at standardised pressure and temperature (e.g. 1 bar and zero degrees Celsius). These equivalent volumetric flow rates can be displayed in addition to or as an alternative to display of the measurement of actual volumetric flow rates.

The measurement correlator 58 preferably performs time integration of the volumetric flow rate measurements (whether actual, standardised, or both) to give total volumetric flow over the period of integration, respectively expressed as actual volume, standardised volume, or both. These total volumetric flows can also be displayed on the display device 60, as can the efficiency of the supply system in terms of the relative volumetric flow rates of the conduits 32 and 48.

The correlator 58 preferably correlates the various measurements of volumetric flow rates and volumetric flows to give indications of the respective performances of the various parts of the system 10 and of the diver 18, plus indications of possible or actual leaks from any part of the system 10, the hoses 12 and 14, and the diver's helmet 16. (Performance of the diver 18 can be monitored by measurement of his respiratory minute volume, which is closely related to his rate of physical exertion, while an abnormally high or low respiratory minute volume may indicate a medical emergency). An abnormally high volumetric flow rate of make-up gases 42 may indicate an unacceptably serious leak.

Fig.2 illustrates a diver's breathing gas mixture supply system which is a modification of the system shown in Fig. 1. Those parts of the system of Fig. 2 which individually correspond to the system of Fig. 1 are given the same reference numeral as in Fig.1, but prefixed by a "1" (i.e. the same reference numeral as in Fig. 1, plus "100"). Since so much of the system of Fig. 2 is structurally and functionally identical to the system of Fig. 1, the following description of Fig. 2 is largely confined to the differences of the system of Fig. 2 relative to the system of Fig. 1; for a detailed description of the system of Fig. 2, reference should be made to the corresponding parts of Fig. 1.

The principal diffference between the system of Fig. 2 and the system of Fig. 1 is that in Fig. 2, the make-up gases from the make-up gas source 142 enter the gas conduit leading to the inlet end of the diver's supply hose 112 upstream of the gas flow conduit 132 within the first gas flow measuring system 130. As in Fig. 1, the volumetric flow rate of the make-up gases are measured by means of the second gas flow measuring system 146. However, the first gas flow measuring system 130 now measures the total volumetric gas flow rate of the gas mixture entering the surface end of the diver's supply hose 112. (In the Fig. 1 system, the first gas flow measuring system 30 measured only the volumetric gas flow rate of the regenerated breathing gas mixture leaving the regenerator 20, and the volumetric gas flow rate of the make-up gases was independently measured by the second gas flow measuring system 46). Consequently, the measurement correlator 158 is suitably modified to take account of the qualitatively different volumetric gas flow rate measurements produced by the first gas flow measuring system 130, which now represent the total supply to the diver 118 (neglecting leakage).

As further possible modifications of the invention, any one or all of the gas analysers 34, 54, 134 and 154 may be replaced by devices that directly measure the specific gravity of the gas mixture flowing in the respective gas conduit to which they are coupled, the measurement correlator 58 and/or 158 being appropriately modified. As an alternative to use of pure helium for calibration of the gas analysers or other devices for directly or indirectly measuring specific gravity, calibration can be carried with any suitable gas mixture of known composition. Mass flowmeters operating on a principle other than measurement of Coriolis forces can be employed in place of any one or all of the mass flowmeters 38, 50, 138 and 150.

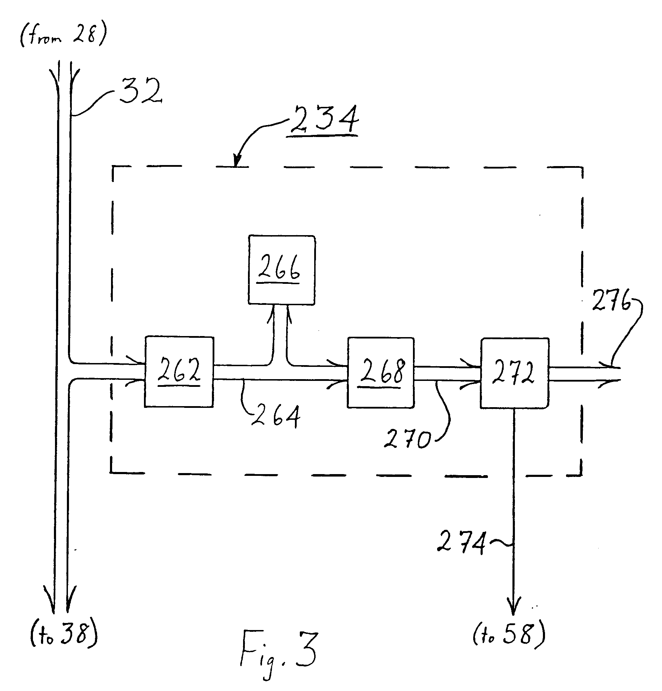

As a still further modification of the present invention, any one or all of the gas analysers 34, 54, 134 and 154 may be replaced by the system shown in Fig. 3. In the first and second embodiments of Figs. 1 and 2, the above referenced gas analysers operated at the hyperbaric internal pressure of the supply system 10 or 110 respectively, and their respective output readings required transformation to give equivalent readings at the standardised pressure of 1 bar. The system of Fig. 3 is an arrangement for obtaining an oxygen content measurement at approximately atmospheric pressure, and hence for obtaining an indirect reading of specific gravity at atmospheric pressure without the previously mentioned transformations of measurement in accordance with actual pressure and temperature (and the additional complexity of measuring such parameters).

Referring now to Fig. 3 in detail, this schematically illustrates a gas analyser circuit 234 intended to be a modification or replacement of the gas analyser circuit 34 of Fig.1. The gas analyser circuit 234 is coupled to the gas flow conduit 32 in like manner to the gas analyser 34 of Fig. 1. Within the circuit 234, gas from the conduit 32 is first reduced in pressure within a pressure regulator 262, which may be constituted by the commercially available "Tescom regulator". The pressure-regulated output from the regulator 262 is delivered along a conduit 264 to a pressure indicator 266 and to a variable-area flowmeter 268. The pressure indicator 266 and the variable-area flowmeter 268 are utilised to set the pressure regulator 262 to deliver an output pressure in the conduit 264 which is just above ambient atmospheric pressure around the supply system 10 when the volume passing through the flowmeter 268 is in the range 1-1.5 litres per minute (which is a small aliquot of the gas mixture flowing through the conduit 32).

The gas passing through the flowmeter 268 is delivered along an output conduit 270 to an oxygen analyser 272 which is preferably in the same form as the analyser 34 (i.e. as in either of US Patents Nos. 3,429,796 and 3,767,552), subject to any necessary or desirable modifications for working at ambient atmospheric pressure in contrast to the previously described hyperbaric pressure. The oxygen-content-representative electrical output signal from the analyser 272 is delivered on an output signal lead 274 which is connected in place of the lead 36 shown in Fig. 1. Gases which have passed through the analyser 272 are exhausted as waste to ambient atmosphere through a vent 276.

The correlator 58 will be modified as necessary to deal with the signal from the substitute gas analyser circuit 234 on the basis that it now represents the oxygen content of a representative sample of the gas mixture flowing along the conduit 32, but already transformed to an equivalent measurement at atmospheric pressure (assumed equal to the standardised pressure). Thus the readings on the display device 60 will now be the measurement of standardised volumetric flow rate and standardised total volumetric flow.

The gas analyser 54 is preferably modified or substituted in like manner to the modification or substitution of the gas analyser circuit 234 for the gas analyser 34. Similar modifications can be made to either or both the gas analysers 134 and 154 in the second embodiment shown in Fig. 2.

The measuring apparatus of the invention thus allows measurement of the volumetric flow rate of a gas mixture in a conduit by measuring the mass flow rate of the mixture and the relative proportions of the gases in the mixture. The gases themselves being known, measurement of their relative proportions allows the specific gravity of the mixture to be determined and hence the volumetric flow rate to be obtained from the mass flow rate. In the examples discussed herein, the measuring apparatus is applied to the supply and make up lines of a closed loop breathing gas mixture supply system, however it will be appreciated that the apparatus can be introduced into the system at any point depending upon the information required. The display device of these examples may also include, or be replaced by, digital displays for volumetric flow rates and efficiency measurements (e.g. in percentage terms), electro-mechanical integrators for monitoring total volumetric flow, hard-copy facilities such as printers on plotters, or digital storage media, etc.

The systems of Figs. 1 and 2 have been described with respect to monitoring the supply and consumption of a diver's breathing gas mixture during diving operations. The gas flow measuring system of the invention is not restricted to such application, and a number of other uses are possible; for example, (1) monitoring the chamber reclamation and purification of spent breathing gases after a diving operation is concluded, such as when the gases are being purified by a process not employed during diving operations, e.g. to remove metabolites other than carbon dioxide; (2) accurate volumetric metering of the delivery of bulk supplies of gases from a gas manufacturer/supplier; (3) monitoring the volumetric flow rates of oxygen-enriched air employed for medical or therapeutic purposes, as in hyperbaric oxygen therapy or to aid the breathing of hospital patients at atmospheric pressure; (4) monitoring the volumetric flow rates of anaesthetic/analgesic gas mixtures, whether in closed-loop breathing systems or in open-loop breathing systems; and (5) monitoring the volumetric flow rates of gas mixtures employed for industrial purposes, e.g. in chemical engineering.

## Claims

1. A gas flow measuring system comprising a gas flow conduit (32; 132) for carrying a flowing gas mixture of known constituents in varying proportions, gas analysing means (34; 134) coupled to the conduit (32; 132) whereby the specific gravity of the flowing gas mixture may be determined, a mass flow meter (38; 138) coupled to the conduit (32; 132) for measuring the mass flow rate of the flowing gas mixture, and a measurement correlator (58; 158) for correlating a currently determined specific gravity of the mixture and the measured mass flow rate to produce a resultant signal (60; 160) representative of the volumetric flow rate of the flowing gas mixture.

2. A gas flow measuring system as claimed in claim 1 characterised in that the gas analysing means (34; 134) measures the proportion of the gas mixture that is constituted by one of two components making up a binary gas mixture.

3. A gas flow measuring system as claimed in claim 2 characterised in that the gas analysing means (34; 134) is an electrochemical oxygen analyser producing an electrical output signal directly from electrochemical reaction of components of the analyser with the oxygen to analyse the oxygen content.

4. A gas flow measuring system as claimed in any preceding claim characterised in that the mass flow meter (38; 138) is a Coriolis-type mass flow meter, which gives a measurement of mass flow rate.

5. A gas flow measuring system as claimed in any preceding claim, characterised in that the measurement correlator (58; 158) derives the volumetric flow rate from the measured mass flow rate and the density of the gas mixture, said density being derived from the specific gravity of the gas mixture which is in turn derived from the measured relative proportions of the gases in the mixture.

6. A gas flow measuring system as claimed in any preceding claim characterised by a pressure regulator (262) coupled between the gas flow conduit (32) and the gas analyser (272) to reduce the pressure of the gas mixture being analysed to a standardised pressure such that the measurement of specific gravity is a measurement at the standardised pressure and the measure of volumetric flow rate at the standardised pressure.

7. A gas flow measuring system as claimed in any preceding claim characterised in that the measurement correlator (58; 158) is adapted to perform a time integration of instantaneous volumetric flow rate to produce a measurement of total volumetric flow over a period of time.

8. A closed loop breathing system incorporating a gas flow measuring system as claimed in any preceding claim, wherein the flowing gas mixture is a breathing gas mixture circulating in the closed-loop breathing system and additional quantities of breathing gas mixture are intermittently or continuously injected into the closed-loop breathing system as make-up gases, and wherein the gas flow measuring system is duplicated (30, 46; 130, 146) for separate volumetric flow measurement of gas mixture flowing around the closed-loop breathing system and of gas mixture injected into the closed-loop breathing system as make-up gases.

9. A closed-loop breathing system as claimed in claim 8, wherein the closed-loop breathing system includes a breathing gas mixture reprocessing means (20), and the gas flow measuring system (30) which measures the volumetric flow rate of gases circulating in the closed loop is connected between the reprocessing means (20) and a breathing gas consumer (18), and wherein the make-up gases (42) are injected through the other gas flow measuring system (46) into the closed-loop at a point between the loop flow measuring system (30) and the consumer (18).

10. A closed-loop breathing system as claimed in claim 8, wherein the closed-loop breathing system includes a breathing gas mixture reprocessing means (120), and the gas flow measuring system (130) which measures the volumetric flow rate of gases circulating in the closed-loop is connected between the reprocessing means (120) and a breathing gas consumer (118), and wherein the make-up gases (142) are injected through the other gas flow measuring system (146) into the closed-loop at a point between the reprocessing means (120) and the loop flow measuring system (130).

## Patentansprüche

1. Gasdurchflußmeßsystem mit einer Gasdurchflußleitung (32; 132), welche eine strömende Gasmischung aus bekannten Bestandteilen in veränderlichen Anteilen führt, mit einer mit der Leitung (32; 132) gekoppelten Analysiereinrichtung (34; 134) zum Bestimmen des spezifischen Gewichts der strömenden Gasmischung, mit einem mit der Leitung (32; 132) gekoppelten Massendurchflußmesser (38; 138) zum Messen des Massendurchsatzes der strömenden Gasmischung, und mit einer Einrichtung (Korrelator 58; 158), welche das laufend bestimmte spezifische Gewicht der Mischung und den gemessenen Massendurchsatz miteinander in Beziehung setzt und daraus ein Signal (60; 160) bildet, welches ein Maß für den volumetrischen Durchsatz der strömenden Gasmischung ist.

2. Gasdurchflußmeßsystem nach Anspruch 1, dadurch gekennzeichnet, dass die Gasanalysiereinrichtung (34; 134) den Anteil einer von zwei Bestandteilen, die eine binäre Gasmischung bilden, an der Gasmischung mißt.

3. Gasdurchflußmeßsystem nach Anspruch 2, dadurch gekennzeichnet, dass die Gasanalysiereinrichtung (34; 134) ein elektrochemischer Sauerstoffanalysator ist, welcher unmittelbar aus der elektrochemischen Reaktion von Bestandteilen des Analysators mit dem Sauerstoff ein elektrisches Ausgangssignal bildet, um den Sauerstoffgehalt zu bestimmen.

4. Gasdurchflußmeßsystem nach einem der vorstehenden Ansprüche, dadurch gekennzeichnet, dass der Massendurchflußmesser (38; 138) ein solcher ist, welcher den Massendurchsatz auf der Grundlage des Corioliseffekts mißt.

5. Gasdurchflußmeßsystem nach einem der vorstehenden Ansprüche, dadurch gekennzeichnet, dass der Korrelator (58; 158) den volumetrischen Durchsatz ableitet aus dem gemessenen Massendurchsatz und aus der Dichte der Gasmischung, welche aus dem spezifischen Gewicht der Gasmischung abgeleitet wird, welches seinerseits aus den gemessenen relativen Anteilen der Gase in der Mischung abgeleitet wird.

6. Gasdurchflußmeßsystem nach einem der vorstehenden Ansprüche, gekennzeichnet durch einen Druckregler (262), welcher zwischen der Gasdurchflußleitung (32) und dem Gasanalysator (272) angeordnet ist und den Druck der zu analysierenden Gasmischung auf einen festgesetzten Druck herabsetzt, so dass die Messung des spezifischen Gewichts eine Messung bei dem festgesetzten Druck ist und sich der volumetrische Durchsatz bezogen auf den festgesetzten Druck ergibt.

7. Gasdurchflußmeßsystem nach einem der vorstehenden Ansprüche, dadurch gekennzeichnet, dass der Korrelator (58; 158) dazu eingerichtet ist, den augenblicklichen volumetrischen Durchsatz über die Zeit zu integrieren und dadurch einen Meßwert des totalen Volumenstroms über einen Zeitabschnitt zu bilden.

8. Atemsystem mit geschlossenem Kreislauf, welches ein Gasdurchflußmeßsystem nach einem der vorstehenden Ansprüche enthält, in welchem die strömende Gasmischung eine Atemgasmischung ist, welche in dem geschlossenen Kreislauf des Atemsystems zirkuliert und in welchem in Zeitabständen oder dauernd zusätzliche Mengen einer Atemgasmischung in den geschlossenen Kreislauf des Atemsystems zur Ergänzung eingeführt werden und in welchem das Gasdurchflußmeßsystem doppelt (30, 46; 130, 146) vorhanden ist, um den Volumendurchsatz der Gasmischung, welche im geschlossenen Kreislauf des Atemsystems zirkuliert, und den Volumendurchsatz der ergänzend in den geschlossenen Kreislauf des Atemsystems eingeführten Gasmischung getrennt zu messen.

9. Atemsystem mit geschlossenem Kreislauf nach Anspruch 8, welches eine Einrichtung (20) zum Wiederaufarbeiten einer Atemgasmischung enthält und in welchem das Gasdurchflußmeßsystem (30), welches den Volumendurchsatz der im geschlossenen Kreislauf zirkulierenden Gase mißt, zwischen der Einrichtung (20) zum Wiederaufarbeiten und einem Atemgasverbraucher (18) angeordnet ist, und in welchem die zur Ergänzung eingeführten Gase (42) durch das andere Gasdurchflußmeßsystem (46) hindurch in den geschlossenen Kreislauf an einer Stelle eingeführt werden, die zwischen dem den Durchfluß im Kreislauf messenden System (30) und dem Verbraucher (18) liegt.

10. Atemsystem mit geschlossenem Kreislauf nach Anspruch 8, welches eine Einrichtung (120) zum Wiederaufarbeiten einer Atemgasmischung enthält und in welchem das Gasdurchflußmeßsystem (130), welches den volumetrischen Durchsatz der im geschlossenen Kreislauf zirkulierenden Gase mißt, zwischen der Einrichtung (120) zum Wiederaufarbeiten und einem Atemgasverbraucher (118) liegt, und in welchem die zur Ergänzung eingeführten Gase (142) durch das andere Gasdurchflußmeßsystem (146) hindurch an einer Stelle in den geschlossenen Kreislauf eingeführt werden, welche zwischen der Einrichtung (120) zum Wiederaufarbeiten und dem System (130) zum Messen des Durchflusses im Kreislauf liegt.

## Revendications

1. Système pour mesurer le débit d'un gaz comprenant un conduit de circulation du gaz (32 ; 132) pour acheminer un mélange circulant dont les constituants connus sont en proportions variables, un moyen analyseur (34 ; 134) accouplé au conduit (32 ; 132) qui permet de déterminer le poids spécifique du mélange de gaz on circulation, un débitmètre massique (38 ; 138) accouplé au conduit (32 ; 132) pour mesurer le débit massique du mélange de gaz en circulation, et un corrélateur de mesure (58 ; 158) pour corréler un poids spécifique du mélange actuellement déterminé avec le débit massique mesuré pour former un signal résultant (60 ; 160) représentatif du débit volumétrique du mélange de gaz en circulation.

2. Système de mesure du débit d'un gaz selon la revendication 1, caractérisé en ce que le moyen analyseur de gaz (34 ; 134) mesure la proportion du mélange de gaz qui est constitué par l'un des deux composants formant un mélange binaire de gaz.

3. Système de mesure du débit d'un gaz selon la revendication 2, caractérisé en ce que le moyen analyseur de gaz (34 ; 134) est un analyseur électrochimique à oxygène produisent un signal de sortie électrique directement à partir de la réaction électrochimique des composante do l'analsyeur avec l'oxygène afin d'analyser la teneur en oxygène.

4. Système de mesure du débit d'un gaz selon l'une quelconque des revendications précédentes, caractérisé en ce que le débitmètre massique (38 ; 138) est un débitmètre massique du type Coriolis qui donne une mesure du débit massique.

5. Système de mesure du débit d'un gaz selon l'une quelconque des revendications précédentes, caractérisé en ce que le corrélateur de mesure (58 ; 158) déduit le débit volumétrique du débit massique mesuré et de la densité du mélange de gaz, ladite densité étant déduite du poids spécifique du mélange de gaz lequel est à son tour déduit des proportions relatives mesurées des gaz dans le mélange.

6. Système de mesure du débit d'un gaz selon l'une quelconque des revendications précédentes, caractérisé en ce qu'un régulateur de pression (262) est accouplé entre le conduit de circulation du gaz (32) et l'analyseur de gaz (272) dans le but de réduire la pression du mélange de gaz à analyser à une pression normalisée de manière que la mesure du poids spécifique soit une mesure sous pression normalisée et que la mesure du débit volumétrique corresponde à la pression normalisée.

7. Système de mesure du débit d'un gaz selon l'une quelconque des revendications précédentes, caractérisé en ce que le corrélateur de mesure (58 ; 158) est conçu pour exécuter une intégration dans le temps du débit volumétrique instantané afin d'obtenir une mesure du débit volumétrique total sur une période de temps donnée.

8. Système respiratoire en boucle fermée incorporant un système de mesure du débit d'un gaz selon l'une quelconque des revendications précédentes, dans lequel le mélange de gaz en circulation est un mélange de gaz respiratoire circulant dans le système respiratoire en boucle fermée, et dans lequel des quantités supplémentaires du mélange de gaz respiratoire sont injectées par intermittence ou en continu dans le système respiratoire en boucle fermée sous forme de gaz d'appoint, et dans lequel le système de mesure du débit de gaz est doublé (30, 46 ; 130, 146) pour mesurer séparément le débit volumétrique du mélange de gaz circulant dans le système respiratoire en boucle fermée et du mélange de gaz injecté dans le système respiratoire en boucle fermée sous forme de gaz d'appoint.

9. Système respiratoire en boucle fermée selon la revendication 8, dans lequel le système respiratoire en boucle fermée comprend un moyen (20) de rotraitement du mélange de gaz respiratoire, et le système de mesure (30) du débit de gaz qui mesure le débit volumétrique des gaz circulent dans la boucle fermée est branché entre le moyen de retraitement (20) et un utilisateur du gaz respiratoire (18), et dans lequel les gaz d'appoint (42) sont injectés à travers l'autre système de mesure du débit de gaz (46) dans la boucle fermée en un point situé entre le système de mesure du débit de la boucle (30) et l'utilisateur (18).

10. Système respiratoire en boucle fermée selon la revendication 8, dans lequel le système respiratoire en boucle fermée comprend un moyen de retraitement du mélange de gaz respiratoire (150), et le système de mesure de débit (130) qui mesure le débit volumétrique des gaz circulant dans la boucle fermée est branché entre le moyen de retraitement (120) et un utilisateur de gaz respiratoire (118), et dans lequel les gaz d'appoint (142) sont injectés à travers le second système de mesure du débit de gaz (146) dans la boucle formée en un point situé entre le moyen de retraitement (120) et le système de mesure du débit de la boucle (130).
